# DEMANDE DE BREVET EUROPEEN

(11) **EP 1 559 774 A1**
(43) Date de publication de la demande: **03.08.2005**
(21) Numéro de dépôt: 04292984.4
(22) Date de dépôt: 14.12.2004
(51) Int. Cl.: C11D 17/00, C11D 3/22, A61K 7/02, C11D 10/04

(54) **Composition de nettoyage de la peau ou des cheveux**

(30) Priorité: 27.01.2004 FR 0450137
(71) Demandeur: L'OREAL, 75008 Paris (FR)
(72) Inventeur: Fonolla Moreno, Angeles, 75013 Paris (FR)
(74) Mandataire: Rasson, Catherine

(57) **Abrégé**

La présente demande concerne une composition de nettoyage comprenant dans un milieu aqueux physiologiquement acceptable (1) un système tensioactif comprenant au moins un tensioactif anionique et au moins un tensioactif amphotère, (2) un système épaississant comprenant au moins deux polymères anioniques comportant au moins une chaîne hydrophobe.

La composition se présente sous forme d'un gel ayant à la fois une viscosité appropriée pour une bonne préhension et de bonnes qualités de mousse.

Le tensioactif anionique est choisi de préférence parmi les sels des hydrolysats de protéines de blé, de soja, d'avoine ou de soie, et le tensioactif amphotère est de préférence une bétaïne.

De préférence, le système épaississant comprend au moins un copolymère comportant au moins un monomère d'acrylates, méthacrylates ou itaconates d'alcool gras en C₁₂-C₃₀ oxyéthyléné, et au moins un copolymère comportant au moins un monomère d'acrylates ou méthacrylates d'acide gras en C₁₂-C₃₀.

Cette composition peut être utilisée notamment dans le domaine cosmétique comme produits de nettoyage ou de démaquillage de la peau, ou comme produits de nettoyage des cheveux.

## Description

La présente invention se rapporte à une composition de nettoyage moussante rinçable comprenant un système tensioactif particulier et un système épaississant particulier, et à l'utilisation de ladite composition, notamment dans le domaine cosmétique comme produits de nettoyage ou de démaquillage de la peau, y compris des paupières, et comme produits de nettoyage des cheveux.

Le nettoyage de la peau est très important pour le soin du visage. Il doit être le plus performant possible car les résidus gras tels que l'excès de sébum, les restes des produits cosmétiques utilisés quotidiennement et les produits de maquillage s'accumulent dans les replis cutanés et peuvent obstruer les pores de la peau et entraîner l'apparition de boutons.

Un moyen pour bien nettoyer la peau est d'utiliser des produits de nettoyage moussants. Les produits de nettoyage moussants actuellement disponibles dans le commerce se présentent sous forme de pains, de gels ou de crèmes moussants, et ils peuvent ou non contenir des savons (sels d'acides gras). Les produits moussants avec savons ont l'avantage de donner une mousse onctueuse ; cependant, certains consommateurs reprochent à ces produits de provoquer des tiraillements dus à leur détergence trop importante. Pour avoir un produit mieux toléré, on cherche à diminuer le taux de savons. Toutefois, le produit a alors une viscosité et un pouvoir moussant insuffisants.

Par ailleurs, les produits moussants sans savons sont généralement bien tolérés. Ils se présentent le plus souvent sous forme de produits liquides ou de gels. Ces produits sont utilisés comme les produits avec savon, c'est-à-dire que l'on applique le produit sur la peau et mouille avec de l'eau pour émulsionner le produit sur la peau, puis on rince à l'eau. Il est connu notamment d'utiliser des gels aqueux moussants plus faciles à manipuler que les produits liquides car ils ne coulent pas. Leur action nettoyante est apportée par les tensioactifs qu'ils contiennent, ces tensioactifs mettant en suspension les résidus gras et les pigments des produits de maquillage. On cherche notamment à faire des gels nettoyants moussants transparents car, tout comme l'eau, la transparence est le symbole de pureté et donc de propreté, et les gels transparents sont ainsi particulièrement appréciés des utilisateurs.

Toutefois, la présence des épaississants dans le gel affecte souvent ses qualités de mousse et induit notamment un mauvais démarrage de la mousse. De plus, après rinçage, la peau est souvent glissante et il ne reste pas la sensation d'une peau propre du fait de la présence d'un résidu filmogène sur la peau, difficile à éliminer.

Ainsi, il subsiste le besoin de produits de nettoyage moussants sans savon, se présentant sous forme de gels, notamment de gels transparents ou translucides, qui aient les propriétés requises pour des produits moussants, à savoir un bon mélange à l'eau, une transformation rapide en mousse et un bon rinçage, tout en ayant la viscosité voulue.

La demanderesse a découvert de manière surprenante que l'utilisation d'un système tensioactif particulier et d'un système épaississant particulier permettait l'obtention de produits moussants, coulant sous leur propre poids tout en étant assez épais, c'est-à-dire ayant la viscosité d'un gel, et ayant de bonnes propriétés moussantes, c'est-à-dire un démarrage de mousse facile et rapide et un bon pouvoir de rinçage lors de l'utilisation. Ces produits sont onctueux et ont de bonnes propriétés cosmétiques.

Ainsi, la présente demande a pour objet une composition de nettoyage comprenant dans un milieu aqueux physiologiquement acceptable (1) un système tensioactif comprenant au moins un tensioactif anionique et au moins un tensioactif amphotère, (2) un système épaississant comprenant au moins deux polymères anioniques comportant au moins une chaîne hydrophobe. La composition peut constituer une composition cosmétique ou dermatologique. Il s'agit de préférence d'une composition cosmétique.

On entend ici par « milieu physiologiquement acceptable » un milieu compatible avec la peau, les muqueuses, le cuir chevelu, les yeux et/ou les cheveux. Par ailleurs, il s'agit d'un milieu aqueux, c'est-à-dire d'un milieu comportant de l'eau. La quantité d'eau est de préférence d'au moins 35 % en poids; elle va de préférence de 35 à 98 % en poids et mieux de 40 à 95 % en poids par rapport au poids total de la composition.

Les compositions de l'invention sont des compositions de nettoyage moussantes et rinçables. Elles se présentent sous forme d'un gel pouvant s'écouler sous son propre poids sans être liquide, c'est-à-dire ayant une viscosité pouvant aller par exemple de 3 à 200 poises (0,3 à 20 Pa.s), et mieux de 5 à 150 poises (0,5 à 15 Pa.s), et encore mieux de 10 à 120 poises (1 à 12 Pa.s), mesurée à 25°C à l'aide du Rheomat RM180 de Rheometric Scientific, cet appareil étant équipé d'un mobile différent selon les viscosités, par exemple d'un mobile 2 pour les gammes de viscosités inférieures à 7 poises, d'un mobile 3 pour les gammes de viscosités de 2 à 40 poises, et d'un mobile 4 pour les gammes de viscosités de 20 poises à 120 poises.

Les compositions selon l'invention ont un pH compatible avec des applications sur la peau, généralement compris entre 4,5 et 7.

Le gel obtenu est généralement transparent ou translucide, ce qui signifie que la composition a une turbidité inférieure ou égale à 500 NTU. Les NTU (Nephelometric Turbidity Units) sont les unités de mesure de la turbidité d'une composition. La mesure de turbidité peut être faite par exemple avec un turbidimètre model 2100P de la société HACH Compagny, les tubes utilisés pour la mesure étant référencés AR397A cat 24347-06. Les mesures sont effectuées à température ambiante (20°C à 25°C). La transparence d'une composition peut se mesurer soit par le coefficient de transmittance à 600 nm soit par la turbidité. La composition de l'invention a un coefficient de transmittance à 600 nm allant de 10 à 90 % ou bien une turbidité allant de 2 à 500 NTU, et de préférence de 5 à 300 NTU.

### Système tensioactif

La composition moussante selon l'invention contient un système tensioactif qui va apporter le caractère moussant à la composition et qui comprend au moins un tensioactif anionique et au moins un tensioactif amphotère. Ces tensioactifs sont des tensioactifs moussants. Ce système peut contenir éventuellement en outre un ou plusieurs tensioactifs moussants non ioniques.

La quantité de système tensioactif, c'est-à-dire la quantité totale de tensioactifs dans la composition, peut aller par exemple, en poids (de matière active) de 0,5 à 40 % en poids, de préférence de 1 à 25 % en poids et mieux de 1 à 20 % en poids par rapport au poids total de la composition.
1. Les tensioactifs anioniques peuvent être choisis notamment parmi les dérivés anioniques de protéines d'origine végétale, les dérivés des aminoacides, les alkyl sulfates, les alkyl éther sulfates, les sulfonates, les iséthionates, les taurates, les sulfosuccinates, les alkyl sulfoacétates, les phosphates et alkylphosphates, les polypeptides, les dérivés anioniques d'alkyl polyglucoside, et leurs mélanges.
   Les dérivés anioniques de protéines d'origine végétale sont des hydrolysats de protéine à groupement hydrophobe, ledit groupement hydrophobe pouvant être naturellement présent dans la protéine ou être ajouté par réaction de la protéine et/ou de l'hydrolysat de protéine avec un composé hydrophobe. Les protéines sont d'origine végétale, et le groupement hydrophobe peut être notamment une chaîne grasse, par exemple une chaîne alkyle comportant de 10 à 22 atomes de carbone.
   Comme dérivés anioniques de protéines d'origine végétale, utilisables dans la composition selon l'invention, on peut plus particulièrement citer les hydrolysats de protéines de blé, de soja, d'avoine ou de soie, comportant une chaîne alkyle ayant de 10 à 22 atomes de carbone et leurs sels. La chaîne alkyle peut être notamment une chaîne lauryle et le sel peut être un sel de sodium, de potassium et/ou d'ammonium. On peut citer par exemple les sels de sodium, de potassium et/ou d'ammonium des hydrolysats de protéine de soie modifiée par l'acide laurique, tels que le produit commercialisé sous la dénomination KAWA SILK par la société Kawaken ; les sels de sodium, de potassium et/ou d'ammonium des hydrolysats de protéine de blé modifiée par l'acide laurique, tels que le sel de potassium commercialisé sous la dénomination AMINOFOAM W OR par la société Croda (nom CTFA : Potassium lauroyl wheat aminoacids) et le sel de sodium commercialisé sous la dénomination PROTEOL LW 30 par la société Seppic (nom CTFA : sodium lauroyl wheat aminoacids) ; les sels de sodium, de potassium et/ou d'ammonium des hydrolysats de protéine d'avoine comportant une chaîne alkyle ayant de 10 à 22 atomes de carbone, et plus spécialement les sels de sodium, de potassium et/ou d'ammonium des hydrolysats de protéine d'avoine modifiée par l'acide laurique, tels que le sel de sodium commercialisé sous la dénomination PROTEOL OAT par la société Seppic (nom CTFA : Sodium lauroyl oat aminoacids).
   Les dérivés des aminoacides peuvent être choisis par exemple parmi les sarcosinates et notamment les acylsarcosinates comme le lauroyl sarcosinate de sodium commercialisé sous la dénomination SARKOSYL NL 97® par la société Ciba ou commercialisé sous la dénomination ORAMIX L 30® par la société Seppic, le myristoyl sarcosinate de sodium, commercialisé sous la dénomination NIKKOL SARCOSINATE MN® par la société Nikkol, le palmitoyl sarcosinate de sodium, commercialisé sous la dénomination NIKKOL SARCOSINATE PN ® par la société Nikkol ; les alaninates comme le N-lauroyl-N-methylamidopropionate de sodium, commercialisé sous la dénomination SODIUM NIKKOL ALANINATE LN 30® par la société Nikkol ou commercialisé sous la dénomination ALANONE ALE®,par la société Kawaken, et le N-lauroyl N-methylalanine triéthanolamine, commercialisé sous la dénomination ALANONE ALTA ® par la société Kawaken ; les N-acylglutamates comme le mono-cocoylglutamate de triéthanolamine commercialisé sous la dénomination ACYLGLUTAMATE CT-12® par la société Ajinomoto, et le lauroyl-glutamate de triéthanolamine commercialisé sous la dénomination ACYLGLUTAMATE LT-12® par la société Ajinomoto ; les aspartates comme le mélange de N-lauroylaspartate de triéthanolamine et de N-myristoylaspartate de triéthanolamine, commercialisé sous la dénomination ASPARACK ® par la société Mitsubishi ; les citrates.
   Comme alkyl éther sulfates, on peut citer par exemple le lauryl éther sulfate de sodium (C12-14 70/30) (2,2 OE) commercialisé sous les dénominations SIPON AOS 225® ou TEXAPON N702 PATE® par la société Cognis, le lauryl éther sulfate d'ammonium (C12-14 70/30) (3 OE) commercialisé sous la dénomination SIPON LEA 370® par la société Cognis, l'alkyl (C12-C14) éther (9 OE) sulfate d'ammonium commercialisé sous la dénomination RHODAPEX AB/20® par la société Rhodia Chimie.
   Comme sulfonates, on peut citer par exemple les alpha-oléfines sulfonates comme l'alpha-oléfine sulfonate de sodium (C14-16) commercialisé sous la dénomination BIO-TERGE AS-40® par la société Stepan, commercialisé sous les dénominations WITCONATE AOS PROTEGE® et SULFRAMINE AOS PH 12® par la société Witco ou commercialisé sous la dénomination BIO-TERGE AS-40 CG® par la société Stepan, l'oléfine sulfonate de sodium secondaire commercialisé sous la dénomination HOSTAPUR SAS 30® par la société Clariant ; les alkyl aryl sulfonates linéaires comme le xylène sulfonate de sodium commercialisé sous les dénominations MANROSOL SXS30®, MANROSOL SXS40®, MANROSOL SXS93® par la société Manro.
   Comme iséthionates, on peut citer les acyliséthionates comme le cocoyl-iséthionate de sodium, tel que le produit commercialisé sous la dénomination JORDAPON CI P® par la société Jordan.
   Comme taurates, on peut citer le sel de sodium de méthyltaurate d'huile de palmiste commercialisé sous la dénomination HOSTAPON CT PATE® par la société Clariant ; les N-acyl N-méthyltaurates comme le N-cocoyl N-methyltaurate de sodium commercialisé sous la dénomination HOSTAPON LT-SF® par la société Clariant ou commercialisé sous la dénomination NIKKOL CMT-30-T® par la société Nikkol, le palmitoyl methyltaurate de sodium commercialisé sous la dénomination NIKKOL PMT® par la société Nikkol.
   Comme sulfosuccinates, on peut citer par exemple le mono-sulfosuccinate d'alcool laurylique (C12/C14 70/30) oxyéthyléné (3 OE) commercialisé sous les dénominations SETACIN 103 SPECIAL®, REWOPOL SB-FA 30 K 4® par la société Witco, le sel di-sodique d'un hemi-sulfosuccinate des alcools C12-C14, commercialisé sous la dénomination SETACIN F SPECIAL PASTE® par la société Zschimmer Schwarz, l'oléamidosulfosuccinate di-sodique oxyéthyléné (2 OE) commercialisé sous la dénomination STANDAPOL SH 135® par la société Cognis, le mono-sulfosuccinate d'amide laurique oxyéthyléné (5 OE) commercialisé sous la dénomination LEBON A-5000® par la société Sanyo, le sel di-sodique de mono-sulfosuccinate de lauryl citrate oxyéthyléné (10 OE) commercialisé sous la dénomination REWOPOL SB CS 50® par la société Witco, le mono-sulfosuccinate de mono-éthanolamide ricinoléique commercialisé sous la dénomination REWODERM S 1333® par la société Witco.
   Comme phosphates et alkylphosphates, on peut citer par exemple les monoalkylphosphates et les dialkyl phosphates, tels que le mono-phosphate de lauryle commercialisé sous la dénomination MAP 20® par la société Kao Chemicals, le sel de potassium de l'acide dodécyl-phosphorique, mélange de mono- et di-ester (diester majoritaire) commercialisé sous la dénomination CRAFOL AP-31® par la société Cognis, le mélange de monoester et de di-ester d'acide octylphosphorique, commercialisé sous la dénomination CRAFOL AP-20® par la société Cognis, le mélange de monoester et de diester d'acide phophorique de 2-butyloctanol éthoxylé (7 moles d'OE), commercialisé sous la dénomination ISOFOL 12 7 EO-PHOSPHATE ESTER@ par la société Condea, le sel de potassium ou de triéthanolamine de mono-alkyl (C12-C13) phosphate commercialisé sous les références ARLATONE MAP 230K-40® et ARLATONE MAP 230T-60® par la société Uniqema, le lauryl phosphate de potassium commercialisé sous la dénomination DERMALCARE MAP XC-99/09® par la société Rhodia Chimie.
   Les dérivés anioniques d'alkyl-polyglucosides peuvent être notamment des citrates, tartrates, sulfosuccinates, carbonates et éthers de glycérol obtenus à partir des alkyl polyglucosides. On peut citer par exemple le sel de sodium d'ester tartrique de cocoylpolyglucoside (1,4), commercialisé sous la dénomination EUCAROL AGE-ET® par la société Cesalpinia, le sel di-sodique d'ester sulfosuccinique de cocoylpolyglucoside (1,4), commercialisé sous la dénomination ESSAI 512 MP® par la société Seppic, le sel de sodium d'ester citrique de cocoyl polyglucoside (1,4) commercialisé sous la dénomination EUCAROL AGE-EC® par la société Cesalpinia.
   Selon un mode préféré de réalisation de l'invention, le système tensioactif contient comme tensioactif anionique, au moins un dérivé anionique de protéines d'origine végétale. Ce tensioactif anionique peut être notamment choisi parmi les sels de sodium, de potassium et/ou d'ammonium des hydrolysats de protéine d'avoine comportant une chaîne alkyle ayant de 10 à 22 atomes de carbone, et plus particulièrement les sels de sodium, de potassium et/ou d'ammonium des hydrolysats de protéine d'avoine modifiée par l'acide laurique, tel que le produit commercialisé sous la dénomination PROTEOL OAT par la société Seppic (nom CTFA : Sodium Lauroyl Oat Amino Acids) (nom CTFA), étant entendu qu'il peut contenir en outre un ou plusieurs autres tensioactifs anioniques.
2. Les tensioactifs amphotères (ce terme incluant les tensioactifs amphotères et zwitterioniques) peuvent être choisis par exemple parmi les bétaïnes, les N-alkylamidobétaïnes et leurs dérivés, les dérivés de la glycine, les sultaïnes, les alkyl polyaminocarboxylates, les alkylamphoacétates et leurs mélanges.
   Comme bétaïnes, on peut citer notamment les alkylbétaïnes comme par exemple la cocobétaïne comme le produit commercialisé sous la dénomination DEHYTON AB-30® par la société Cognis, la laurylbétaïne comme le produit commercialisé sous la dénomination GENAGEN KB® par la société Clariant, la laurylbétaïne oxyethylénée (10 OE), comme le produit commercialisé sous la dénomination LAURYLETHER(10 OE)BETAINE® par la société Shin Nihon Rica, la stéarylbétaïne oxyéthylénée (10 OE) comme le produit commercialisé sous la dénomination STEARYLETHER(10 OE)BETAINE® par la société Shin Nihon
   Rica.
   Parmi les N-alkylamidobétaines et leurs dérivés, on peut citer par exemple la cocamidopropyl bétaine commercialisée sous la dénomination LEBON 2000 HG® par la société Sanyo, ou commercialisée sous la dénomination EMPIGEN BB® par la société Albright & Wilson, la lauramidopropyl bétaïne commercialisée sous la dénomination REWOTERIC AMB12P® par la société Witco.
   Comme dérivés de la glycine, on peut citer le N-cocoylglycinate de sodium commercialisé sous la dénomination AMILITE GCS-12® par la société Ajinomoto.
   Comme sultaines, on peut citer le cocoyl-amidopropylhydroxy-sulfobetaine commercialisé sous la dénomination CROSULTAINE C-50® par la société Croda.
   Come alkyl polyaminocarboxylates (APAC), on peut citer le cocoylpolyamino-carboxylate de sodium, commercialisé sous la dénomination AMPHOLAK 7 CX/C®,et AMPHOLAK 7 CX® par la société Akzo Nobel, le stéaryl-polyamidocarboxylate de sodium commercialisé sous la dénomination AMPHOLAK 7 TX/C par la société Akzo Nobel, la carboxyméthyloléyl-polypropylamine de sodium, commercialisé sous la dénomination AMPHOLAK X07/C® par la société Akzo Nobel.
   Comme alkylamphoacétates, on peut citer par exemple le N-cocoyl-N-carboxyméthoxyéthyl-N-carboxyméthyl-éthylènediamine N-di-sodique (nom CTFA : disodium cocamphodiacetate) comme le produit commercialisé sous la dénomination MIRANOL C2M CONCENTRE NP® par la société Rhodia Chimie, et le N-cocoyl-N-hydroxyéthyl-N-carboxyméthyl-éthylènediamine N-sodique (nom CTFA : sodium cocamphoacetate).
   Selon un mode préféré de réalisation de l'invention, le système tensioactif contient comme tensioactif amphotère, au moins une bétaïne, et notamment une alkylbétaïne telle que la cocobétaïne, étant entendu qu'il peut contenir en outre un ou plusieurs autres tensioactifs amphotères.
3. Comme indiqué ci-dessus, le système tensioactif utilisé dans la composition de l'invention peut contenir en outre un ou plusieurs tensioactifs non ioniques. Ces tensioactifs non ioniques peuvent être choisis par exemple parmi les alkyl polyglucosides (APG), les esters de maltose, les alcools gras polyglycérolés, les dérivés de glucamine comme l'éthyl-2 hexyl oxy-carbonyl n-méthyl glucamine, et leurs mélanges.

Comme alkylpolyglucosides, on utilise de préférence ceux contenant un groupe alkyle comportant de 6 à 30 atomes de carbone et de préférence de 8 à 16 atomes de carbone, et contenant un groupe hydrophile (glucoside) comprenant de préférence 1,2 à 3 unités de saccharide. Comme alkylpolyglucosides, on peut citer par exemple le decylglucoside (Alkyl-C9/C11-polyglucoside (1.4)) comme le produit commercialisé sous la dénomination MYDOL 10 ® par la société Kao Chemicals, le produit commercialisé sous la dénomination PLANTAREN 2000 UP ® par la société Cognis, et le produit commercialisé sous la dénomination ORAMIX NS 10 ® par la société Seppic ; le caprylyl/capryl glucoside comme le produit commercialisé sous la dénomination ORAMIX CG 110 ® par la Société Seppic ; le laurylglucoside comme les produits commercialisés sous les dénominations PLANTAREN 1200 N ® et PLANTACARE 1200 ® par la société Cognis ; et le coco-glucoside comme le produit commercialisé sous la dénomination PLANTACARE 818/UP ® par la société Cognis.

Les dérivés de maltose sont par exemple ceux décrits dans le document EP-A-566438, tels que l'O-octanoyl-6'-D-maltose, ou encore le O-dodecanoyl-6'-D-maltose décrit dans le document FR-2,739,556.

Parmi les alcools gras polyglycérolés on peut citer le dodecanediol polyglycérolé (3,5 moles de glycérol), produit commercialisé sous la dénomination CHIMEXANE NF® par la société Chimex.

Selon un mode préféré de réalisation de l'invention, le tensioactif non ionique éventuellement présent est choisi de préférence parmi les alkylpolyglucosides.

Quand la composition contient des tensioactifs non ioniques, la quantité de tensioactifs non ioniques (en matière active) peut aller par exemple de 0,5 à 20 % en poids et mieux de 0,5 à 15 % en poids par rapport au poids total de la composition.

### Système épaississant

Le système épaississant comprend au moins deux polymères anioniques comportant au moins une chaîne hydrophobe, ces polymères anioniques étant différents l'un de l'autre.

La ou les chaînes hydrophobes du polymère anionique utilisé selon l'invention sont notamment des chaînes hydrocarbonées linéaires ou ramifiées, saturées ou insaturées, ayant de 12 à 30 atomes de carbone, telles que alkyle, arylalkyle, alkylaryle, alcoylène ; des groupements divalents cycloaliphatiques tels que notamment méthylènedicyclohexyl et isophorone ; ou des groupements divalents aromatiques tels que phénylène.

Les polymères anioniques utilisés dans la présente invention peuvent être choisis notamment parmi les copolymères d'acide carboxylique à insaturation α,β-monoéthylénique, notamment d'acide acrylique ou méthacrylique. On entend par « copolymères » aussi bien les copolymères obtenus à partir de deux sortes de monomères que ceux obtenus à partir de plus de deux sortes de monomères tels que les terpolymères obtenus à partir de trois sortes de monomères.

Les polymères anioniques utilisés de préférence dans l'invention sont des copolymères obtenus par copolymérisation d'un ou plusieurs monomères (a) choisis parmi les acides carboxyliques à insaturation α,β-éthylénique ou leurs esters, avec un monomère (b) à insaturation éthylénique comportant un groupement hydrophobe.

L'acide carboxylique à insaturation α,β-monoéthylénique constituant le monomère (a) peut être choisi parmi de nombreux acides et en particulier parmi l'acide acrylique, l'acide méthacrylique, l'acide crotonique, l'acide itaconique, l'acide maléique, et leurs esters. Il s'agit de préférence de l'acide acrylique ou de l'acide méthacrylique, et leurs esters.

Le monomère (b) comportant un groupement hydrophobe peut être en particulier choisi parmi les acrylates, méthacrylates ou itaconates d'alcool gras en C₁₂-C₃₀ oxyéthyléné (1 à 50 OE), tels que le steareth-20 méthacrylate, le méthacrylate de béhényle oxyéthyléné (25 OE), l'itaconate de mono-cétyle oxyéthyléné (20 OE), l'itaconate de mono-stéaryle oxyéthyléné (20 OE), l'acrylate modifié par des alcools en C12-C24 polyoxyéthylénés (25 OE), et parmi les acrylates ou méthacrylates d'acide gras en C₁₂-C₃₀ tels que les acrylates ou méthacrylates de décyle, de lauryle, de stéaryle, de béhényle ou de mélissyle, et leurs mélanges.

Comme polymères anioniques comportant au moins un monomère d'acrylates, méthacrylates ou itaconates d'alcool gras en C₁₂-C₃₀ oxyéthyléné, on peut citer notamment le copolymère acrylates/steareth-20 methacrylate tel quel ou sous forme de dispersion aqueuse, comme le produit commercialisé sous la dénomination Aculyn 22 par la société Rohm & Haas (nom CTFA : Acrylates/Steareth-30 Methacrylate copolymer) ; le terpolymère acide (méth)acrylique / acrylate d'éthyle / méthacrylate de béhényle oxyéthyléné (25 OE), tel que le produit en émulsion aqueuse commercialisé sous la dénomination Aculyn 28 par la société Rohm & Haas ; le copolymère acide acrylique/itaconate de mono-cétyle oxyéthyléné (20 OE), tel que le produit en dispersion aqueuse à 30 % commercialisé sous la dénomination Structure 3001 par la société National Starch ; le copolymère acide acrylique/itaconate de mono-stéaryle oxyéthyléné (20 OE) tel que le produit en dispersion aqueuse à 30 % commercialisé sous la dénomination Structure 2001 par la société National Starch ; le copolymère acrylates/acrylate modifié par des alcools en C12-C24 polyoxyéthylénés (25 OE), tel que le latex à 30-32 % de copolymère, commercialisé sous la dénomination Synthalen W2000 par la société 3V SA.

Comme polymères anioniques comportant au moins un monomère d'acrylates ou méthacrylates d'acide gras en C₁₂-C₃₀, on peut citer plus particulièrement les polymères comportant un monomère d'acrylates ou méthacrylates de décyle, de lauryle, de stéaryle, de béhényle ou de mélissyle, plus particulièrement acrylates et méthacrylates de stéaryle, et notamment les copolymères vendus sous les noms PEMULEN ou CARBOPOL par la Société Noveon, comme le copolymère acrylate/C₁₀-C₃₀-alkylacrylate tel que les produits PEMULEN TR1, PEMULEN TR2 ou CARBOPOL 1382 (nom CTFA : Acrylates/C10-30 Alkyl acrylate Crosspolymer).

Selon un mode préféré de réalisation de l'invention, les polymères anioniques sont choisis parmi les copolymères comportant au moins un monomère d'acrylates, méthacrylates ou itaconates d'alcool gras en C₁₂-C₃₀ oxyéthyléné, les copolymères comportant au moins un monomère d'acrylates ou méthacrylates d'acide gras en C₁₂-C₃₀, et leurs mélanges (1). Préférentiellement, le système épaississant comprend au moins un copolymère comportant au moins un monomère d'acrylates, méthacrylates ou itaconates d'alcool gras en C₁₂-C₃₀ oxyéthyléné, notamment le copolymère acrylates/steareth-20 methacrylate, et (2) au moins un copolymère comportant au moins un monomère d'acrylates ou méthacrylates d'acide gras en C₁₂-C₃₀, notamment d'acrylates ou méthacrylates de stéaryle. De manière préférée, le système épaississant comprend le copolymère acrylates/steareth-20 methacrylate (Aculyn 22) et le copolymère acrylates/C₁₀-C₃₀-alkylacrylates (PEMULEN TR1, PEMULEN TR2 ou CARBOPOL 1382).

Selon l'invention, le système épaississant peut représenter, en matière active, de 0,05 à 10 % en poids, de préférence de 0,1 à 5 % en poids, mieux de 0,5 à 5 % en poids par rapport au poids total de la composition. De préférence, chacun des polymères est présent en une quantité de 0,01 à 9,99 % en poids, de préférence de 0,1 à 4,5 % en poids par rapport au poids totale de la composition.

La composition selon l'invention peut en outre contenir un ou plusieurs polymères amphotères, tels que les terpolymères acide acrylique / méthylacrylate / chlorure de méthacrylamido-propyltrimonium, comme le POLYQUATERNIUM 47 (nom CTFA) et par exemple les produits commercialisés sous les dénominations MERQUAT 2001, MERQUAT 280, MERQUAT 295 et MERQUAT PLUS 3330 par la société CALGON, ou bien un ou plusieurs polymères cationiques tels que le copolymère de chlorure de diméthyldiallylammonium et d'acrylamide (nom CTFA : Polyquaternium 7) et par exemple les produits commercialisés sous les dénominations MERQUAT S, MERQUAT 2200 et MERQUAT 550 par la société Calgon ou le produit commercialisé sous la dénomination SALCARE SC 10 par la société Ciba. Quand ils sont présents, la quantité de polymères amphotères et/ou cationiques peut aller par exemple, en matière active, de 0,01 à 5 % en poids et de préférence de 0,02 à 3 % en poids par rapport au poids total de la composition.

Le milieu aqueux de la composition selon l'invention peut contenir, outre l'eau, un ou plusieurs solvants choisis parmi les alcools inférieurs (mono-alcools) comportant de 1 à 6 atomes de carbone, tels que l'éthanol ; et les polyols. Comme polyols, on peut citer la glycérine ; les glycols comme le butylène glycol, l'isoprène glycol, le propylène glycol, les polyéthylène glycols tels que le PEG-8 ; le sorbitol ;les sucres tels que le glucose, le fructose, le maltose, le lactose, le sucrose ; et leurs mélanges. La quantité de solvant(s) dans la composition de l'invention peut aller par exemple de 0,1 à 20 % en poids et de préférence de 0,5 à 15 % en poids et mieux de 0,5 à 10 % en poids par rapport au poids total de la composition.

Les compositions de l'invention peuvent contenir des adjuvants habituellement utilisés dans le domaine cosmétique, et notamment ceux utilisés dans les produits de nettoyage. Comme adjuvants, on peut citer par exemple les parfums, les conservateurs, les séquestrants (EDTA), les pigments, les charges notamment exfoliantes, les colorants solubles, les filtres solaires, les actifs cosmétiques ou dermatologiques tels que les hydratants comme l'acide hyaluronique ; les céramides ; les vitamines hydrosolubles ou liposolubles comme la vitamine C et ses dérivés tels que la vitamine CG ; les antiseptiques ; les antiséborrhéïques ; les antimicrobiens tels que le peroxyde de benzoyle, l'acide salicylique, le triclosan, l'acide azélaïque ; les azurants optiques. Les quantités de ces différents adjuvants sont celles classiquement utilisées dans le domaine considéré, et par exemple de 0,01 à 20 % du poids total de la composition. Ces adjuvants ainsi que leurs concentrations doivent être tels qu'ils ne modifient pas la propriété recherchée pour la composition de l'invention.

Les compositions selon l'invention peuvent constituer notamment des produits de nettoyage ou de démaquillage de la peau (corps, visage y compris paupières et cils des yeux), et/ou de nettoyage des cheveux.

Un autre objet de l'invention consiste en l'utilisation cosmétique de la composition telle que définie ci-dessus, comme produit de nettoyage et/ou de démaquillage de la peau ou comme produit de nettoyage des cheveux.

Un autre objet de l'invention consiste en un procédé cosmétique de nettoyage de la peau, caractérisé par le fait qu'on applique la composition de l'invention, sur la peau, en présence d'eau, et qu'on élimine la mousse formée et les résidus de salissure par rinçage à l'eau.

Les exemples qui suivent servent à illustrer l'invention sans toutefois présenter un caractère limitatif. Les quantités indiquées sont en % en poids sauf mention contraire et correspondent à la quantité de matière première et non à la quantité de matière active. Les noms des matières premières sont le plus souvent indiqués en nom CTFA.

### Exemples 1 à 4 selon l'invention et exemple 5 comparatif

| Compositions | Exemple 1 selon l'invention | Exemple 2 selon l'invention | Exemple 3 selon l'invention | Exemple 4 selon l'invention | Exemple 5 comparatif |
|---|---|---|---|---|---|
| Eau | Qsp 100 | Qsp 100 | Qsp 100 | Qsp 100 | Qsp 100 |
| Acrylates/Steareth-30 Methacrylate copolymer (1) | 1 | 2,5 | 0,5 | 3,5 | 1 |
| Acrylates/C10-30 Alkyl acrylate Crosspolymer (2) | 0,3 | 0,4 | 0,5 | 0,4 | 0,3 |
| Triéthanolamine | 0,5 | 0,65 | 0,7 | 0.6 | 0,5 |
| Coco-bétaïne(3) | 3 | 3 | 5 | 3.5 | 3 |
| Sodium Lauroyl Oat Amino Acids (4) | 7 | 1,5 | 2 | 3 | 0 |
| Vitamine CG(5) | 0,5 | 0,2 | 0,5 | 0,2 | 0,5 |
| Ammonium Lauryl sulfate (6) | 11 | 10 | 9 | 8,5 | 11 |
| TEA Lauryl Sulfate(7) | 2,5 | 2 | 3 | 4 | 2,5 |
| Cocoglucoside(8) | 2,5 | 1,5 | 1,5 | 3 | 2,5 |
| Decylglucoside (9) | 1,5 | 2 | 1,5 | 2,5 | 1,5 |
| Glycérine | 4 | 3 | 4 | 2,5 | 4 |
| Polyquaternium 47 (10) | 0,5 | 0,5 | 0 | 0,3 | 0,5 |
| Disodium EDTA | 0,2 | 0,2 | 0,2 | 0,2 | 0,2 |
| Extrait de menthe (11) | 0,3 | 0,4 | 0,6 | 0,5 | 0,3 |
| Parfum | 0,3 | 0,3 | 0,3 | 0,3 | 0,3 |
| Viscosité | 2,2 Pa.s (mobile 3) | 7,3 Pa.s (mobile 4) | Non mesurée | 10 Pa.s (mobile 4) | Inférieure à 0,3 Pa.s |

| | | | | | |
|---|---|---|---|---|---|
| (1) Aculyn 22 | | | | | |
| (2) Carbopol 1382 | | | | | |
| (3) Dehyton AB 30 de la société Cognis (solution aqueuse à 30 % de matière active) | | | | | |
| (4) Proteol OAT (solution aqueuse à 30 % de matière active) | | | | | |
| (5) Ascorbyl glucoside | | | | | |
| (6) Empicol AL30/FL de la société Huntsman (solution aqueuse à 30 % de matière active) | | | | | |
| (7) Texapon T42 de la société Cognis (solution aqueuse à 40 % de matière active) | | | | | |
| (8) Plantacare 818UP de la société Cognis (solution aqueuse à 53 % de matière active) | | | | | |
| (9) Mydol 10 de la société KAO (solution aqueuse à 40 % de matière active) | | | | | |
| (10) Merquat 2001 de la société Ondeo (solution aqueuse à 20 % de matière active) | | | | | |
| (11) Calmiskin (Mentha piperita) de la société Silab (extrait en solution aqueuse) | | | | | |

Les compositions des exemples décrits ci-dessus présentent un pH de 5,8 à 6,3 ± 0,3. Elles ont un aspect nacré, transparent et lisse. Les exemples 1 à 4 présentent un bon démarrage de mousse, donnent une mousse assez dense et ont un bon pouvoir nettoyant. L'exemple 5 a une viscosité insuffisante, ce qui entraîne un moins bon confort d'utilisation, et cet exemple montre qu'il est indispensable d'avoir à la fois le système tensioactif et le système épaississant conformes à l'invention pour obtenir une composition ayant les propriétés de viscosité et de qualité de mousse requises.

Sur un panel de 38 personnes qui ont testé la composition de l'exemple 1, 85 % ont trouvé que la mousse était suffisante, qu'elle était de qualité satisfaisante et que le produit était facile à rincer, 82 % ont trouvé que le produit était doux et 94 % ont trouvé que le produit était agréable à utiliser.

### Exemple 6 selon l'invention et exemples comparatifs 7 et 8

| | Exemple 6 selon l'invention | Exemple 7 comparatif | Exemple 8 comparatif |
|---|---|---|---|
| Eau | Qsp 100 | Qsp 100 | Qsp 100 |
| Glycérine | 4 | 4 | 4 |
| Methylparaben | 0,3 | 0,3 | 0,3 |
| Disodium EDTA | 0,25 | 0,25 | 0,25 |
| Sodium Lauroyl Oat Amino Acids (1) | 2 | 2 | 2 |
| Ammonium lauryl sulfate (2) | 8 | 8 | 8 |
| TEA Lauryl Sulfate (3) | 2 | 2 | 2 |
| parfum | 0,2 | 0,2 | 0,2 |
| menthol | 0,05 | 0,05 | 0,05 |
| Decylglucoside(4) | 2 | 2 | 2 |
| Cocoglucoside(5) | 2 | 2 | 2 |
| Cocobetaine (6) | 4,5 | 4,5 | 4,5 |
| Acrylates/Steareth-30 Methacrylate copolymer (7) | 2,5 | 2,5 | 2,5 |
| Acrylates/C10-30 Alkyl acrylate | 0,3 | - | - |
| Crosspolymer (8) | | | |
| Polyacrylamide / C13-14 Isoparaffin / Laureth-7 (9) | - | 0,3 | 0,3 |
| Triéthanolamine | 0,2 | 0,2 | 0,5 |
| Aspect | Composition gélifiée | Composition liquide | Composition gélifiée |
| Viscosités à 25°C (mesurée au Rhéomat 180) | 3 Pa.s (30 poises) | 1,3 Pa.s (13 poises) | 3,1 Pa.s (31 poises) |

| | | | |
|---|---|---|---|
| (1) Proteol OAT (solution aqueuse à 30 % de matière active) | | | |
| (2) Empicol AL30/FL de la société Huntsman (solution aqueuse à 30 % de matière active) | | | |
| (3) Texapon T42 de la société Cognis (solution aqueuse à 40 % de matière active) | | | |
| (4) Mydol 10 de la société KAO (solution aqueuse à 40 % de matière active) | | | |
| (5) Plantacare 818UP de la société Cognis (solution aqueuse à 53 % de matière active) | | | |
| (6) Dehyton AB 30 de la société Cognis (solution aqueuse à 30 % de matière active) | | | |
| (7) Aculyn 22 | | | |
| (8) Carbopol 1382 | | | |
| (9) Sepigel 305 (de la société SEPPIC) (à 40 % en matière active) | | | |

L'exemple 6 selon l'invention présente une viscosité supérieure à l'exemple comparatif 7 qui ne contient qu'un seul polymère anionique comportant une chaîne hydrophobe, polymère qui est associé à un polymère anionique ne comportant pas de chaîne hydrophobe (Sepigel 305). L'exemple 7 comparatif est trop liquide et a une viscosité insuffisante, ce qui entraîne un moins bon confort d'utilisation.

En outre, les qualités de l'exemple 6 selon l'invention sont supérieures à celles de l'exemple comparatif 7 car cet exemple 6 présente un meilleur démarrage de la mousse, il donne une mousse beaucoup plus dense et plus abondante, et il se rince très facilement. Au contraire, le démarrage de la mousse est difficile avec l'exemple comparatif 7, et la mousse obtenue n'est ni dense, ni abondante.

L'exemple comparatif 8 qui contient une plus grande quantité de triéthanolamine que l'exemple 7 a une viscosité comparable à celle de l'exemple 6 mais son pH est passé à 7 au lieu de 6,3 pour les exemples 6 et 7, ce qui est désavantageux pour une utilisation sur la peau. En outre, ses qualités d'utilisation sont semblables à celles de l'exemple 7, donc bien inférieures à celles de l'exemple 6 selon l'invention.

## Revendications

1. Composition de nettoyage comprenant dans un milieu aqueux physiologiquement acceptable, (1) un système tensioactif comprenant au moins un tensioactif anionique et au moins un tensioactif amphotère, (2) un système épaississant comprenant au moins deux polymères anioniques comportant au moins une chaîne hydrophobe.

2. Composition selon la revendication 1, **caractérisée en ce qu'**elle comprend de 35 à 98 % en poids d'eau par rapport au poids total de la composition.

3. Composition selon la revendication 1 ou 2, **caractérisée en ce qu'**elle présente une viscosité allant de 0,3 à 20 Pa.s.

4. Composition selon l'une quelconque des revendications précédentes, **caractérisée en ce que** la quantité de système tensioactif, va de 0,5 à 40 % en poids par rapport au poids total de la composition.

5. Composition selon l'une quelconque des revendications précédentes, **caractérisée en ce que** le tensioactif anionique est choisi parmi les dérivés anioniques de protéines d'origine végétale, les dérivés des aminoacides, les alkyl sulfates, les alkyl éther sulfates, les sulfonates, les iséthionates, les taurates, les sulfosuccinates, les alkyl sulfoacétates, les phosphates et alkylphosphates, les polypeptides, les dérivés anioniques d'alkyl polyglucoside, et leurs mélanges.

6. Composition selon l'une quelconque des revendications précédentes, **caractérisée en ce que** le tensioactif anionique est choisi parmi les hydrolysats de protéines de blé, de soja, d'avoine ou de soie, comportant une chaîne alkyle ayant de 10 à 22 atomes de carbone et leurs sels.

7. Composition selon l'une quelconque des revendications précédentes, **caractérisée en ce que** le tensioactif anionique est choisi parmi les sels de sodium, de potassium et/ou d'ammonium des hydrolysats de protéine d'avoine comportant une chaîne alkyle ayant de 10 à 22 atomes de carbone.

8. Composition selon l'une quelconque des revendications précédentes, **caractérisée en ce que** le tensioactif amphotère est choisi parmi les bétaïnes, les N-alkylamidobétaïnes et leurs dérivés, les dérivés de la glycine, les sultaïnes, les alkyl polyaminocarboxylates, les alkylamphoacétates et leurs mélanges.

9. Composition selon l'une quelconque des revendications précédentes, **caractérisée en ce que** le tensioactif amphotère est la cocobétaïne.

10. Composition selon l'une quelconque des revendications précédentes, **caractérisée en ce que** le système tensioactif comprend en outre un ou plusieurs tensioactifs non ioniques.

11. Composition selon la revendication précédente, **caractérisée en ce que** le tensioactif non ionique est choisi parmi les alkylpolyglucosides.

12. Composition selon l'une quelconque des revendications précédentes, **caractérisée en ce que** les polymères anioniques sont choisis parmi les copolymères d'acide carboxylique à insaturation α,β-monoéthylénique.

13. Composition selon l'une quelconque des revendications précédentes, **caractérisée en ce que** les polymères anioniques sont choisis parmi les copolymères comportant au moins un monomère d'acrylates, méthacrylates ou itaconates d'alcool gras en C₁₂-C₃₀ oxyéthyléné, les copolymères comportant au moins un monomère d'acrylates ou méthacrylates d'acide gras en C₁₂-C₃₀, et leurs mélanges.

14. Composition selon l'une quelconque des revendications précédentes, **caractérisée en ce que** le système épaississant comprend au moins un copolymère comportant au moins un monomère de type acrylates, méthacrylates ou itaconates d'alcool gras en C₁₂-C₃₀ oxyéthyléné, et au moins un copolymère comportant au moins un monomère de type acrylates et méthacrylates d'acide gras en C₁₂-C₃₀.

15. Composition selon l'une quelconque des revendications précédentes, **caractérisée en ce que** le système épaississant comprend le copolymère acrylates/steareth-20 methacrylate et le copolymère acrylates/C10-C30-alkylacrylates.

16. Composition selon l'une quelconque des revendications précédentes, **caractérisée en ce que** la quantité du système épaississant va de 0,05 à 10 % en poids, de préférence de 0,1 à 5 % en poids par rapport au poids total de la composition.

17. Composition selon l'une quelconque des revendications précédentes, **caractérisée en ce qu'**elle constitue une composition cosmétique.

18. Utilisation cosmétique d'une composition cosmétique selon l'une quelconque des revendications précédentes, comme produit de nettoyage et/ou de démaquillage de la peau ou comme produit de nettoyage des cheveux.

19. Procédé cosmétique de nettoyage de la peau, **caractérisé en ce qu'**on applique la composition selon l'une quelconque des revendications 1 à 17, sur la peau en présence d'eau, et qu'on élimine la mousse formée et les résidus de salissure par rinçage à l'eau.
